# EUROPEAN PATENT SPECIFICATION

(11) **EP 0 748 221 B1**
(45) Date of publication and mention of the grant of the patent: **14.04.1999**
(21) Application number: 95900492.0
(22) Date of filing: 31.10.1994
(51) Int. Cl.: A61K 31/435, C07D 221/02, C07J 73/00

(54) **7-SUBSTITUTED-4-AZA-STEROID DERIVATIVES AS 5-ALPHA- REDUCTASE INHIBITORS**
7-SUBSTITUIERTE-4-AZA-STEROID-DERIVATE ALS 5-ALPHA-REDUCTASE-HEMMER
DERIVES DE 4-AZA-STEROIDE SUBSTITUE EN POSITION 7 UTILISES COMME INHIBITEURS DE 5-ALPHA-REDUCTASE

(30) Priority: 04.11.1993 US 147808
(43) Date of publication of application: 18.12.1996
(73) Proprietor: Merck & Co., Inc., Rahway New Jersey 07065-0900 (US)
(72) Inventor: BAKSHI, Raman, K., Edison, NJ 08820 (US); PATEL, Gool, F., Califon, NJ 07830 (US); RASMUSSON, Gary, H., Watchung, NJ 07060 (US)
(74) Representative: Thompson, John Dr.
(86) International application number: US9412516
(87) International publication number: WO9512398

(56) References cited:
- EP-A- 0 484 094
- EP-A- 0 547 691
- WO-A-92/16233
- WO-A-93/23420
- US-A- 4 377 584
- US-A- 5 215 894
- US-A- 5 237 064
- JOURNAL OF MEDICINAL CHEMISTRY, vol. 29, no. 11, 1 November 1986, pages 2298-2315, XP000568779 RASMUSSON G H ET AL: "AZASTEROIDS: STRUCTURE-ACTIVITY RELATIONSHIPS FOR INHIBITION OF 5ALPHA-REDUCTASE AND OF ANDROGEN RECEPTOR BINDING"
- STEROIDS: STRUCTURE, FUNCTION, AND REGULATION, vol. 47, no. 1, January 1986, MA US, pages 1-19, XP002014015 J. R. BROOKS ET AL: "5.alpha.-Reductase Inhibitory Activity and Anti-Androgenic Activities of Some 4-Azasteroids in The Rat"
- J . STEROID BIOCHEM. MOL. BIOL., vol. 44, no. 2, 1993, pages 121-131, XP002014016 T. N. MELLIN ET AL: "Azasteroids as Inhibitors of Testosterone 5-alpha-Reductase in Mammalian Skin"
- JOURNAL OF CLINICAL ENDO, AND METAB., Vol. 74, No. 2, issued 1992, DIANI et al., "Hair Growth Effects of Oral Administration of Finasteride, Steroid 5 Alpha-Reductase Inhibitor, Alone and in Combination with Topical Minoxidil in the Balding Stumptail Macaque", pages 345-350.

## Description

This application is a continuation-in-part of pending International application number PCT/US93/04643, filed on May 14, 1993, which in turn was a continuation-in-part of U.S. application serial no. 886,572, (Merck case no. 18670) filed May 20, 1992, and now abandoned.

### FIELD OF THE INVENTION

The present invention provides novel compounds, novel compositions, methods of their use and methods of their manufacture, where such compounds are generally pharmacologically useful as agents in therapies whose mechanism of action rely on the inhibition of 5α-reductase, and more particularly, the dual inhibition of the 5α-reductase 1 and 5α-reductase 2 isozymes.

### BACKGROUND OF THE INVENTION

Certain undesirable physiological manifestations, such as acne vulgaris, seborrhea, female hirsutism, androgenic alopecia which includes female and male pattern baldness, and benign prostatic hyperplasia, are the result of hyperandrogenic stimulation caused by an excessive accumulation of testosterone ("T") or similar androgenic hormones in the metabolic system. Early attempts to provide a chemotherapeutic agent to counter the undesirable results of hyperandrogenicity resulted in the discovery of several steroidal antiandrogens having undesirable hormonal activities of their own. The estrogens, for example, not only counteract the effect of the androgens but have a feminizing effect as well. Non-steroidal antiandrogens have also been developed, for example, 4'-nitro-3'-trifluoromethyl-isobutyranilide. See Neri, *et al*., *Endocrinol*. **1972**, *91* (2). However, these products, though devoid of hormonal effects, compete with all natural androgens for receptor sites, and hence have a tendency to feminize a male host or the male fetus of a female host and/or initiate feed-back effects which would cause hyperstimulation of the testes.

The principal mediator of androgenic activity in some target organs, e.g. the prostate, is 5α-dihydrotestosterone ("DHT"), formed locally in the target organ by the action of testosterone-5α-reductase (or simply 5α-reductase). Inhibitors of 5α-reductase will serve to prevent or lessen symptoms of hyperandrogenic stimulation in these organs. See especially United States Patent Nos. 4,377,584, issued March 22, 1983, and 4,760,071, issued July 26, 1988, both assigned to Merck & Co., Inc. It is now known that a second 5α-reductase isozyme exists, which interacts with skin tissues, especially in scalp tissues. See, e.g., G. Harris, *et al*., *Proc. Natl. Acad. Sci. USA, Vol. 89*, pp. 10787-10791 (**Nov. 1992**). The isozyme that principally interacts in skin tissues is conventionally designated as 5α-reductase 1 (or 5α-reductase type 1), while the isozyme that principally interacts within the prostatic tissues is designated as 5α-reductase 2 (or 5α-reductase type 2).

In the treatment of hyperandrogenic disease conditions, e.g. benign prostatic hyperplasia (BPH) and/or the prevention and treatment of prostatic cancer, and the treatment of prostatitis, it would be desirable to have one drug entity which is active against both isozymes to significantly inhibit dihydrotesterone production. It would also be desirable to have one drug entity that is active as a dual inhibitor of both isozymes for the treatment of conditions of the skin and scalp, e.g. acne vulgaris, seborrhea, female hirsutism, and androgenic alopecia. Additionally, such a dual inhibitor of 5α-reductase 1 and 2 could be used in combination with a 5α-reductase 1 inhibitor or with a 5α-reductase 2 inhibitor, e.g. finasteride (PROSCAR®), for combination therapy in the treatment of hyperandrogenic conditions. The dual isozyme inhibitor could also be used in combination with a potassium channel opener, e.g. minoxidil, for the treatment of male pattern baldness. The present invention addresses this by providing compounds that are active as dual 5α-reductase 1 and 2 inhibitors.

### SUMMARY OF THE INVENTION

The novel compounds of the present invention are those of structural Formula I: 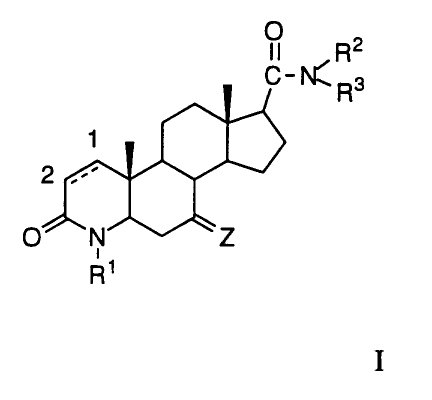 or a pharmaceutically acceptable salt or ester thereof, and are dual inhibitors of 5α-reductase isozymes 1 and 2. The compounds of Formula I are useful in the oral, systemic, parenteral or topical treatment of acne vulgaris, androgenic alopecia including female and male pattern baldness, benign prostatic hyperplasia, female hirsutism, prostatitis, and the prevention and treatment of prostatic carcinoma.

Therefore it is an object of this invention to provide compounds that have sufficient activity in the inhibition of both 5α-reductase isozymes. It is an additional object of this invention to provide methods of using the compounds of Formula I for the treatment and/or prevention of hyperandrogenic conditions such as BPH, female hirsutism, male pattern baldness, acne vulgaris, androgenic alopecia, and prostatic cancer, as well as prostatitis. It is a further object of this invention to provide pharmaceutical compositions for the compounds of Formula I. Another object of this invention is to provide compounds of Formula I in combination with a 5α-reductase 2 inhibitor, such as finasteride, or a 5α-reductase 1 inhibitor, or a potassium channel opener, such as minoxidil, wherein such combinations would be useful in one or more of the above-mentioned methods of treatment or pharmaceutical compositions.

### DETAILED DESCRIPTION OF THE INVENTION

The novel compounds of this invention have the structural Formula I: 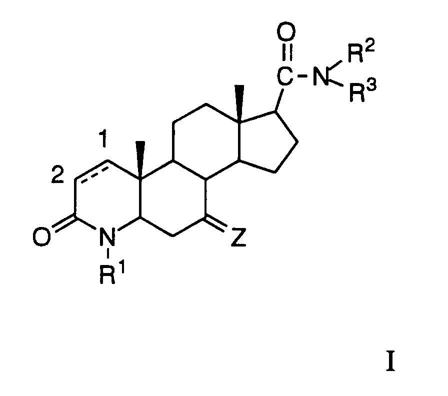 or a pharmaceutically acceptable salt or ester thereof, wherein: the C₁-C₂ bond designated "--------" represents a single or double bond;
R¹ is selected from:
   1) -H,
   2) -CH₃ and
   3) -CH₂CH₃;
Z is selected from:
   1) oxo,
   2) α-H and a β-substituent selected from:
      a) C₁₋₄ alkyl,
      b) C₂₋₄ alkenyl,
      c) -CH₂ COOH,
      d) -OH,
      e) -COOH,
      f) -COO (C₁₋₄ alkyl),
      g) -OCONR⁴R⁵ wherein
         R⁴ and R⁵ are independently selected from
         i) -H,
         ii) -C₁₋₄ alkyl,
         iii) phenyl and
         iv) benzyl;
            or R⁴ and R⁵ taken together with the nitrogen to which they are attached represent a 5-6 membered saturated heterocycle optionally containing one other heteroatom selected from -O-, -NH- and -S-;
      h) C₁₋₄ alkoxy,
      i) C₃₋₆ cycloalkoxy,
      j) -OC(O)R⁷, wherein R⁷ is C₁₋₆alkyl or phenyl,
      k) halo,
      l) halo-C₁₋₂ alkyl,
      m) -CF₃, and
      n) C₃₋₆ cycloalkyl;
   3) =CHR⁶;
   4) spirocyclopropane either unsubstituted or substituted with R⁶;
R² is selected from -H and C₁₋₆ alkyl, either unsubstituted or substituted with one or more of aryl, heteroaryl, -COOH or -OH;
R³ is -C₁₋₆ alkyl substituted with one or more of aryl, heteroaryl, -COOH, -OH or di-aryl amino; and
R⁶ is selected from -H and C₁₋₄ alkyl.

Combinations of substituents and/or variables are permissible only if such combinations result in stable compounds.

In one embodiment of the instant invention are compounds of Formula I wherein R¹ is hydrogen or methyl; R² is hydrogen, methyl, ethyl or propyl; Z is an alpha hydrogen and a beta substituent selected from C₁₋₄ alkyl, C₂₋₄ alkenyl, C₁₋₄ alkoxy, halo-C₁₋₂ alkyl, -CF₃, and C₃₋₆ cycloalkyl; and R³ is C₁₋₄ alkyl disubstituted with phenyl, wherein each phenyl ring is independently unsubstituted or substituted.

In one class of this embodiment are compounds of Formula I that have structural Formula II: 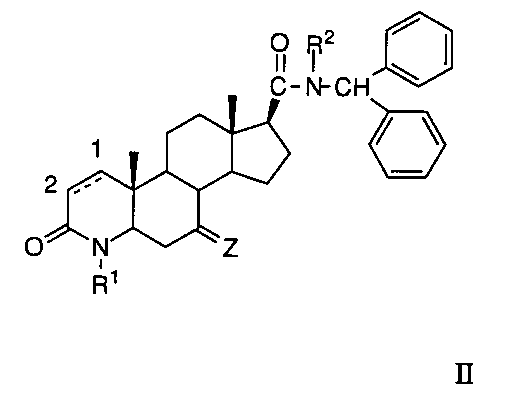 or a pharmaceutically acceptable salt thereof wherein
R¹ is hydrogen or methyl; R² is hydrogen or methyl, and Z is alpha hydrogen and beta methyl.

Some examples of compounds within this class are:
N-(diphenylmethyl)-3-oxo-4-aza-7β-methyl-5α-androstane-17β-carboxamide;
N-(diphenylmethyl)-3-oxo-4-aza-7β-methyl-5α-androst-1-ene-17β-carboxamide;
N-(diphenylmethyl)-3-oxo-4-aza-4,7β-dimethyl-5α-androstane-17β-carboxamide; and
N-(methyl),N-(diphenylmethyl)-3-oxo-4-aza-4,7β-dimethyl-5α-androstane-17β-carboxamide.

As used herein "alkyl" is intended to include both branched- and straight-chain saturated aliphatic hydrocarbon groups having the specified number of carbon atoms, e.g., methyl (Me), ethyl (Et), propyl, butyl, iso-propyl (i-Pr), iso-butyl (i-Bu), sec-butyl (s-Bu), tert-butyl (t-Bu). "Alkyloxy" (or "alkoxy") represents an alkyl group having the indicated number of carbon atoms attached through an oxygen bridge, e.g., methoxy, ethoxy, propyloxy, iso-propoxy, n-butoxy, iso-butoxy, sec-butoxy, t-butoxy and the like. "Alkenyl" is intended to include hydrocarbon groups having the specified number of carbon atoms of either a straight or branched configuration with one or more carbon-carbon double bonds which may occur in any stable point along the chain, such as ethenyl, allyl, 1-propen-1-yl, 1-propen-2-yl, 1-buten-1-yl, 1-buten-2-yl, pentenyl, and the like. Included in this invention are all E, Z diasteriomers.

The term "C₃-C₆ cycloalkyl" as used herein is meant to include, e.g., cyclopropyl, cyclobutyl, cyclopentyl, and cyclohexyl. The term C₃-C₆ cycloalkoxy" as used herein is meant to include a cycloalkyl group attached through an oxygen bridge, e.g., cyclopropyloxy, cyclobutyloxy, cyclopentyloxy, and cyclohexyloxy.

Representative examples of Z are where the a-substituent (dashed lines) is hydrogen and the b-substituent (wedge) is e.g. methyl, ethyl, propyl, allyl, carboxymethyl, hydroxy, methoxy, ethoxy, cyclopropyloxy, cyclopentyloxy, acetoxy, fluoro, chloro, bromo, trifluoromethyl, trichloromethyl, fluoromethyl, chloromethyl, carboxy, N,N-dimethylcarbamoyl, hydroxymethyl, methoxymethyl, and the like. Representative examples where Z is an alkenyl substituent, =CH-R⁶, include, e.g. =CH₂, =CH-CH₃, =CH-CH₂CH₃, and the like.

Representative examples where Z is spirocyclopropane include: 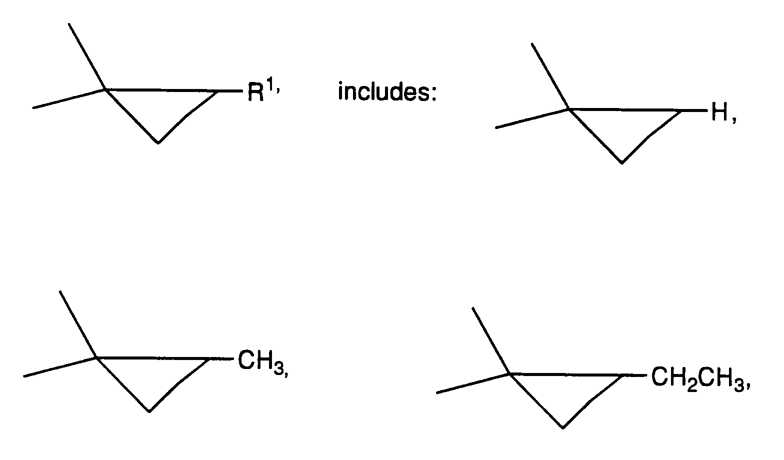 stereoisomers thereof and the like.

Unless otherwise indicated the 17-position substituent is assumed to be in the beta configuration.

As used herein the term "aryl" is intended to include phenyl or 1- or 2-naphthyl, either unsubstituted or mono- or disubstituted. The term "halo" is intended to include chloro, fluoro, bromo and iodo.

The phrase "R⁴ and R⁵ taken together with the nitrogen to which they are attached represent a 5- to 6-membered saturated heterocycle optionally containing one other heteroatom selected from N, -O-, -NH- and -S-" is intended to include ring groups such as N-piperidinyl, N-morpholinyl, N-piperazinyl, N-(4-methyl)piperazinyl, N-thiomorpholinyl, N-pyrrolidinyl, N-imidazolidinyl and the like.

The term "heteroaryl" is intended to include the following either unsubstituted or mono- or di-substituted: pyridyl, furyl, pyrryl, thienyl, isothiazolyl, imidazolyl, benzimidazolyl, tetrazolyl, pyrazinyl, pyrimidyl, quinolyl, isoquinolyl, benzofuryl, isobenzofuryl, benzothienyl, pyrazolyl, indolyl, isoindolyl, purinyl, carbazolyl, isoxazolyl, thiazolyl, oxazolyl, benzthiazolyl, and benzoxazolyl. The heteroaryl ring may be attached within structural Formula I or substituted at any heteroatom (N, O or S) or carbon atom in the ring which results in the creation of a stable structure.

The substituents on the aryl and heteroaryl groups named above are independently selected from: C₁₋₆ alkyl, C₂₋₆ alkenyl, phenyl, halo such as chloro, bromo, iodo and fluoro, trifluoromethyl, cyano, carboxy, C₁₋₆ alkyloxycarbonyl, hydroxy, C₁₋₆ alkoxy, C₁₋₆ alkylcarbonyloxy, C₁₋₆ alkylthio, C₁₋₆ alkylsulfinyl, C₁₋₆ alkylsulfonyl, C₁₋₆ alkylcarbonyl, amino, C₁₋₆ alkylamino, di(C₁₋₆ alkyl)amino, C₁₋₆ alkylcarbonylamino, C₁₋₆ alkyloxycarbonylamino, C₁₋₆ alkylsulfonyl-amino and C₁₋₆ alkylaminosulfonyl.

Whenever the terms "alkyl", "alkenyl", "alkyloxy (or alkoxy)", "aryl" or "heteroaryl", or one of their prefix roots, appear in a name of a substituent in Formula I, (e.g. aralkoxyaryloxy) they shall have the same definitions as those described above for "alkyl", "alkenyl", "alkyloxy (or alkoxy)", "aryl" and "heteroaryl", respectively. Designated numbers of carbon atoms (e.g. C₁₋₁₀) shall refer independently to the number of carbon atoms in an alkyl or alkenyl moiety or to the alkyl or alkenyl portion of a larger substituent in which alkyl or alkenyl appears as its prefix root.

Also included within the scope of this invention are pharmaceutically acceptable salts of the compounds of Formula I, where a basic or acidic group is present on the structure. When an acidic substituent is present, i.e. -COOH, there can be formed the ammonium, sodium, potassium, calcium salt, and the like, for use as the dosage form. Where a basic group is present, i.e. amino or a basic heteroaryl radical such as, e.g., 4-pyridyl, an acidic salt, i.e. hydrochloride, hydrobromide, acetate, pamoate, and the like, can be used as the dosage form.

Also, in the case of the -COOH group being present, pharmaceutically acceptable esters can be employed, e.g. acetate, maleate, pivaloyloxymethyl, and the like, and those esters known in the art for modifying solubility or hydrolysis characteristics for use as sustained release or prodrug formulations.

Representative salts include the following salts: acetate, lactobionate, benzenesulfonate, laurate, benzoate, malate, bicarbonate, maleate, bisulfate, mandelate, bitartrate, mesylate, borate, methylbromide, bromide, methylnitrate, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, pamoate (embonate), estolate, palmitate, esylate, pantothenate, fumarate, phosphate/diphosphate, gluceptate, polygalacturonate, gluconate, salicylate, glutamate, stearate, glycollylarsanilate, sulfate, hexylresorcinate, subacetate, hydrabamine, succinate, hydrobromide, tannate, hydrochloride, tartrate, hydroxynaphthoate, teoclate, Iodide, tosylate, isothionate, triethiodide, lactate, and valerate.

In addition, some of the compounds of the instant invention may form solvates with water or common organic solvents. Such solvates are encompassed within the scope of this invention.

Some of the crystalline forms for compounds of the present invention may exist as polymorphs and as such are intended to be included in the present invention.

The term "therapeutically effective amount" shall mean that amount of a drug or pharmaceutical agent that will elicit the biological or medical response of a tissue, system, animal or human that is being sought by a researcher, veterinarian, medical doctor or other clinician.

The compounds of the present invention are effective in treating the hyperandrogenic conditions of androgenic alopecia including male pattern baldness, acne vulgaris, seborrhea, and female hirsutism by oral, systemic, parenteral or topical administration thereof either alone or in combination with a 5α-reductase 2 inhibitor and/or a 5α-reductase 1 inhibitor and/or a potassium channel opener. The term "treating androgenic alopecia" is intended to include the arresting and/or reversing of androgenic alopecia, and the promotion of hair growth. The compounds of the present invention are also effective in treating benign prostatic hyperplasia, prostatitis, and treating and/or preventing prostatic carcinoma by oral, systemic or parenteral administration thereof either alone or in combination with a 5α-reductase 2 inhibitor and/or in combination with a 5α-reductase 1 inhibitor.

The present invention also has the objective of providing suitable topical, oral, systemic and parenteral pharmaceutical formulations for use in the novel methods of treatment of the present invention. The compositions containing the present compounds as the active ingredient for use in the treatment of the above-noted conditions can be administered in a wide variety of therapeutic dosage forms in conventional vehicles for systemic administration. For example, the compounds can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound desired can be employed as an antiandrogenic agent.

The daily dosage of the products may be varied over a wide range from 0.01 to 1,000 mg per adult human/per day. For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0002 mg/kg to about 50 mg/kg of body weight per day. The range is more particularly from about 0.001 mg/kg to 7 mg/kg of body weight per day.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For the treatment of androgenic alopecia including male pattern baldness, acne vulgaris, seborrhea, and female hirsutism, the compounds of the present invention may be administered in a pharmaceutical composition comprising the active compound in combination with a pharmaceutically acceptable carrier adapted for topical administration. Topical pharmaceutical compositions may be, e.g., in the form of a solution, cream, ointment, gel, lotion, shampoo or aerosol formulation adapted for application to the skin. These topical pharmaceutical compositions containing the compounds of the present invention ordinarily include about 0.001% to 15% by weight of the active compound in admixture with a pharmaceutically acceptable vehicle.

For the treatment of acne vulgaris, androgenic alopecia including male pattern baldness, seborrhea, female hirsutism, benign prostatic hyperplasia, prostatitis and the prevention and/or treatment of prostatic cancer, the compounds of the instant invention can be combined with a therapeutically effective amount of a 5α-reductase 2 inhibitor, such as finasteride, or a 5α-reductase 1 inhibitor, such as 4,7β-dimethyl-4-aza-5α-cholestan-3-one, in a single oral, systemic, or parenteral pharmaceutical dosage formulation. Alternatively, a combined therapy can be employed wherein the compound of Formula I and the 5α-reductase 1 or 2 inhibitor are administered in separate oral, systemic, or parenteral dosage formulations. Also, for the skin and scalp related disorders of acne vulgaris, androgenic alopecia including male pattern baldness, seborrhea, and female hirsutism, the compounds of the instant invention and a 5α-reductase 1 or 2 inhibitor can be formulated for topical administration. For example, a compound of Formula I and finasteride can be administered in a single oral or topical dosage formulation, or each active agent can be administered in a separate dosage formulation, e.g., in separate oral dosage formulations, or an oral dosage formulation of finasteride in combination with a topical dosage formulation of a compound of Formula I. See, e.g., U.S. Patent No.'s 4,377,584 and 4,760,071 which describe dosages and formulations for 5α-reductase inhibitors.

Furthermore, administration of a compound of the present invention in combination with a therapeutically effective amount of a potassium channel opener, such as minoxidil, cromakalin, pinacidil, a compound selected from the classes of S-triazine, thiane-1-oxide, benzopyran, and pyridinopyran derivatives or a pharmaceutically acceptable salt thereof, may be used for the treatment of androgenic alopecia including male pattern baldness. The active agents can be administered in a single topical dosage formulation, or each active agent can be administered in a separate dosage formulation, e.g., in separate topical dosage formulations, or an oral dosage formulation of a compound of Formula I in combination with a topical dosage formulation of, e.g., minoxidil. See, e.g., U.S. Patent No.'s 4,596,812, 4,139,619 and WO 92/02225, published 20 February 1992, for dosages and formulations of calcium channel openers.

For combination treatment with more than one active agent, where the active agents are in separate dosage formulations, the active agents can be administered concomitantly, or they each can be administered at separately staggered times.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound thereof employed. A physician or veterinarian of ordinary skill can readily determine and prescribe the effective amount of the drug required to prevent, counter or arrest the progress of the condition. Optimal precision in achieving concentration of drug within the range that yields efficacy without toxicity requires a regimen based on the kinetics of the drug's availability to target sites. This involves a consideration of the distribution, equilibrium, and elimination of a drug.

In the compositions of the present invention, the compounds herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methyl-cellulose and the like. Other dispersing agents which may be employed include glycerin and the like. For parenteral administration, sterile suspensions and solutions are desired. Isotonic preparations which generally contain suitable preservatives are employed when intravenous administration is desired.

Topical preparations containing the active drug component can be admixed with a variety of carrier materials well known in the art, such as, e.g., alcohols, aloe vera gel, allantoin, glycerine, vitamin A and E oils, mineral oil, PPG2 myristyl propionate, and the like, to form, e.g., alcoholic solutions, topical cleansers, cleansing creams, skin gels, skin lotions, and shampoos in cream or gel formulations. See, e.g., EP 0 285 382.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug camers. Such polymers can include polyvinylpyrrolidone, pyran copolymer, polyhydroxypropylmethacrylamidephenol, polyhydroxy-ethylaspartamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

The compounds of the present invention can be prepared readily according to the following Examples or modifications thereof using readily available starting materials, reagents and conventional synthesis procedures. In these reactions, it is also possible to make use of variants which are themselves known to those of ordinary skill in this art, but are not mentioned in greater detail. The examples are not intended to be limitations on the scope of the instant invention in any way, and they should not be so construed. Furthermore, the compounds described in the following examples are not to be construed as forming the only genus that is considered as the invention, and any combination of the compounds or their moieties may itself form a genus. Those skilled in the art will readily understand that known variations of the conditions and processes of the following preparative procedures can be used to prepare these compounds. All temperatures are in degrees Celsius unless noted otherwise.

### EXAMPLE 1

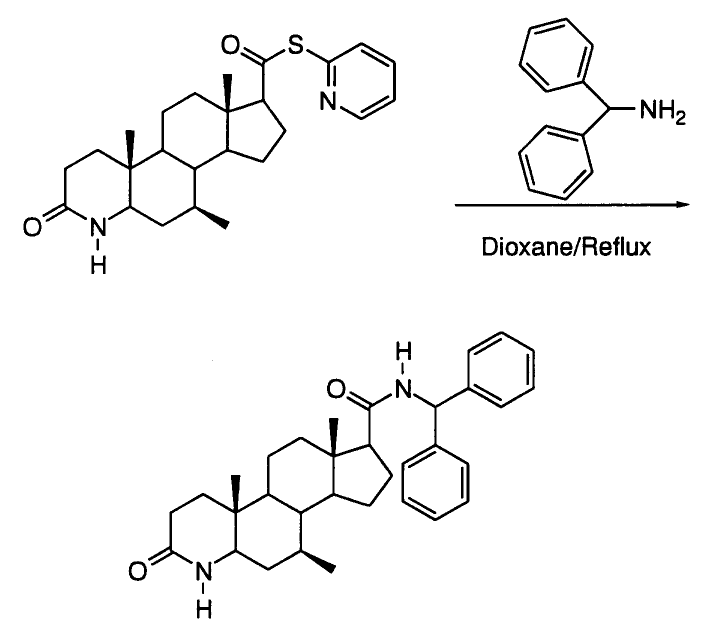

### N-Diphenylmethyl-3-oxo-4-aza-7β-methyl-5α-androstane-17β-carboxamide

To a solution of S-2'-pyridyl-3-oxo-4-aza-7β-methyl-5α-androstane-17β-thiocarboxylate (600 mg, 1.406 mmol) in dioxane (20 ml) was added amino diphenylmethane (512 mg, 2.79 mmol). After stirring the reaction mixture for overnight at 80°, the reaction mixture was concentrated and partitioned between ethyl acetate and water. The organic layer was washed with brine, dried and concentrated. The residue was purified by preparatative TLC (thin layer chromatography) to afford pure product; mp 261-263°. Mass spec. (MS) M+ calculated, 498; observed m/e 499 (m+1) (FAB); ¹H NMR (CDCl₃, 400 MHz, Key Peaks) δ(ppm) 0.666 (s), 0.8358 (s), 0.9975 (d, J=5.9 Hz), 3.051 (dd, J=3.9 Hz, J=11.85 Hz).

### EXAMPLE 2

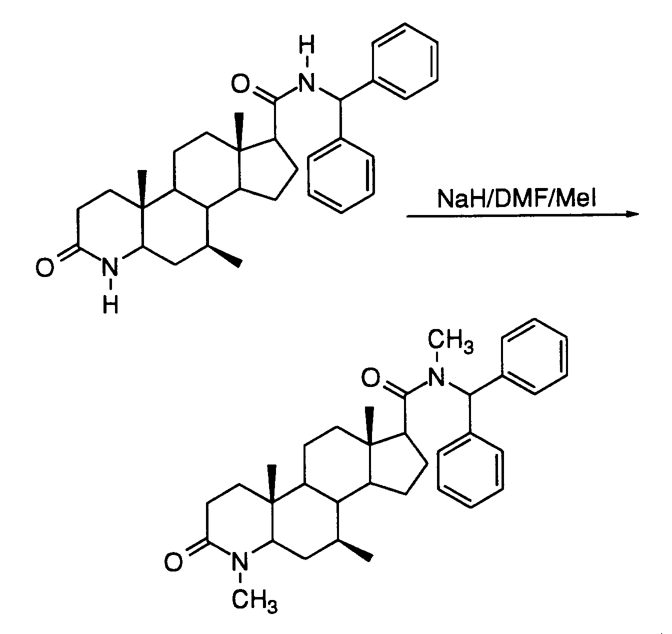

### N-Diphenylmethyl-N-methyl-3-oxo-4-aza-4,7β-dimethyl-5α-androstane-17β-carboxamide

To a solution of N-diphenylmethyl-3-oxo-4-aza-7β-methyl-5α-androstane-17β-carboxamide (70 mg, 0.14 mmol) in 1 ml of N,N-dimethylformamide (DMF) was added sodium hydride (40 mg, 50% suspension). After stirring the reaction mixture for 15 min., methyl iodide (200 µl) was added. The reaction mixture was allowed to stir for overnight at room temperature, quenched with water and extracted with ethyl acetate. The organic layer was washed with water, brine, dried, concentrated and purified by prep. TLC to give pure product. Mass spec. (MS) M+ calculated, 526; observed m/e 527 (m+1) (FAB): ¹H NMR (CDCl₃, 400 MHz, Key Peaks) δ(ppm) 0.814 (s), 0.840 (s), 1.056 (d, J=6.06 Hz), 3.02 (dd, J=3.22 Hz, J=12.45 Hz).

### EXAMPLE 3

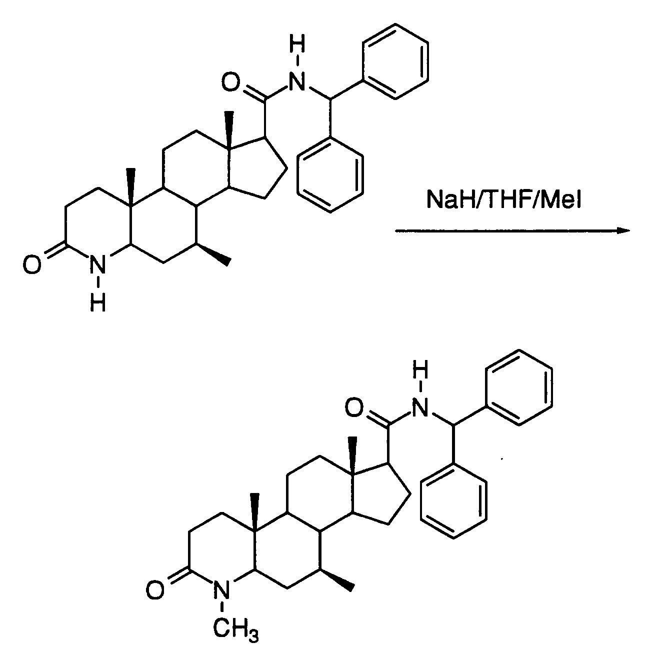

### N-Diphenylmethyl-3-oxo-4-aza-4,7β-dimethyl-5α-androstane-17β-carboxamide

To a solution of N-diphenylmethyl-3-oxo-4-aza-7β-methyl-5α-androstan-17β-carboxamide (70 mg, .14 mmol) in 1 ml of tetrahydrofuran (THF) was added sodium hydride (40 mg, 50% suspension). After stirring the reaction mixture for 15 min., methyl iodide (200 µl) was added. The reaction mixture was allowed to stir for overnight at room temperature, quenched with water and extracted with ethyl acetate. The organic layer was washed with water, brine, dried, concentrated and purified by prep. TLC to give pure product; mp 118-120°. Mass spec. (MS) M+ calculated, 512; observed m/e 513 (m+1) (FAB); ¹H NMR (CDCl₃, 400 MHz, Key Peaks) δ(ppm) 0.659 (s), 0.815 (s), 1.025 (d, J=6.1 Hz), 3.01 (dd, J=3.38 Hz, J=12.73 Hz).

### EXAMPLE 4

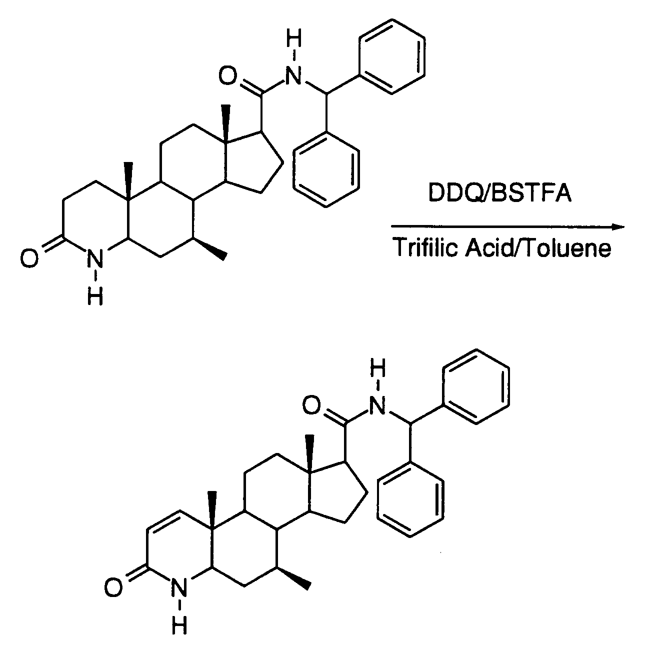

### N-Diphenylmethyl-3-oxo-4-aza-7β-methyl-5α-androst-1-ene-17β-carboxamide

To a solution of N-diphenylmethyl-3-oxo-4-aza-7β-methyl-5α-androstan-17β-carboxamide (200 mg, .402 mmol) in toluene (10 ml) was added bis(trimethylsilyl)trifluoroacetamide (BSTFA) (454 mg, 1.768 mmol), trifilic acid (trifluoromethanesulfonic acid)(4 µl) and dichlorodicyano benzoquinone (DDQ) (122 mg, 0.442 mmol). After stirring the reaction mixture overnight at room temperature, methyl acetoacetate (9.3 ml, 0.08 mmol) was added and reaction mixture refluxed for 6 hrs. The mixture was cooled to 23°, diluted with ethyl acetate, washed with aq., sodium carbonate, aq., sodium bisulfite, brine, dried and concentrated. Crude product was purified by prep. TLC to afford pure product; mp 142-144°. Mass spec. (MS) M+ calculated, 496; observed m/e 497 (m+1) (FAB); ¹H NMR (CDCl₃, 400 MHz, Key Peaks) δ(ppm) 0.68 (s), 0.89 (s), 1.01 (d, J=5.72 Hz), 3.32 (dd, J=3.72 Hz, J=12.36 Hz).

### EXAMPLE 5

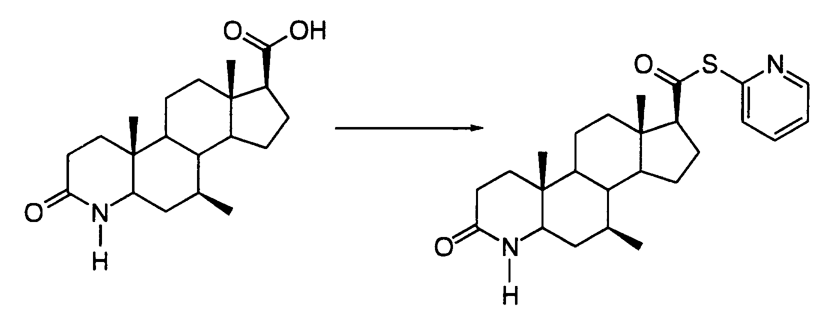

### [S-(2-Pyridyl)-7β-methyl-3-oxo-4-aza-5α-androstane-17β-thiocarboxylate

To a solution of 7β-methyl-3-oxo-4-aza-5α-androstane-17β-carboxylic acid (1.3 g, 3.9 mmol) in toluene (20 ml) was added aldrithiol (1.7 g, 7.8 mmol) and triphenylphosphine (2.04 g, 7.8 mmol). After stirring at room temperature overnight the reaction mixture was eluted through 200 ml of silica gel with 10% acetone in methylene chloride to give crystalline product. Recrystallization from ethyl acetate afforded the title compound.

### EXAMPLE 6

### Preparation of 7β-methyl-3-oxo-4-aza-5α-androstane-17β-carboxylic acid

As seen below in Flowsheets A and B, pregnenolone-3-acetate P is first reduced to the alcohol II by sodium borohydride in ethanol at -10 to 0°C. The alcohol II is then protected by a dimethyl-t-butyl silyl (TBS) group in DMF with TBS chloride and imidazole as a base at room temperature. The protected alcohol is then oxidized to the corresponding 5-en-7-one III by treatment with hydrogen t-butyl peroxide and chromium hexacarbonyl in e.g., acetonitrile, at reflux. The 7-methyl group can be introduced at this point by a Grignard reaction using e.g., methyl magnesium chloride in e.g., anhydrous THF at 0 to -10°C to produce the 7-methyl-7-hydroxy adduct IV. This is then oxidized with e.g. aluminum isopropoxide and cyclohexanone (Oppenauer oxidation conditions) in refluxing toluene solvent to produce the 7-methyl-4,6-dien-3-one V. This in turn is reduced via e.g., metal-ammonia, THF and toluene at -78°C to selectively yield the 7-beta-methyl-5-en-3-one VI. In the next step the delta-5 double bond is isomerized to the 4-ene by use of DBU (1,8-diazabicyclo[5.4.0] undec-7-ene) in e.g. refluxing tetrahydrofuran (THF) to produce the 7-methyl-4-ene-3-one, VII. The A ring is next cleaved by treatment with e.g. potassium permanganate, sodium periodate in t-butyl alcohol at 80°C to produce the corresponding seco-acid VIII.

Treatment of the seco-acid with ammonium acetate in glacial acetic acid at 120°C yields e.g., the 7-methyl-4-aza-pregn-5-en-3-one IX. This in turn is selectively reduced with e.g., PtO₂, to remove the 5-double bond to produce the 5α-hydrogen compound X. The TBS protecting group is next removed by aqueous HF in acetonitrile at room temperature and then oxidized by tetrapropylammonium perruthenate/4-methylmorpholine N-oxide in methylene chloride at room temperature to yield the 17-acetyl compound XI. This is treated with a sodium hypobromite/sodium hydroxide solution in dioxane at 10-15°C to form the title 17-carboxylic acid XII, mp. 311-312°C. This is then used as described above to make the 2-thiopyridyl ester and the resulting azasteroidal amides.

The 1,2-double bond in the A ring can be introduced into XII by DDQ oxidation (see procedure in U.S. Patent 5,084,574) to produce XIII, mp. 328-330°C. Formation of the 2-thiopyridyl intermediate XIV, analogously as described above, and reaction with benzhydrylamines as described above produces the corresponding amides. In the schemes below, "Ac" represents an acetyl group and "Ar" represents an aryl group.

### FLOWSHEET A

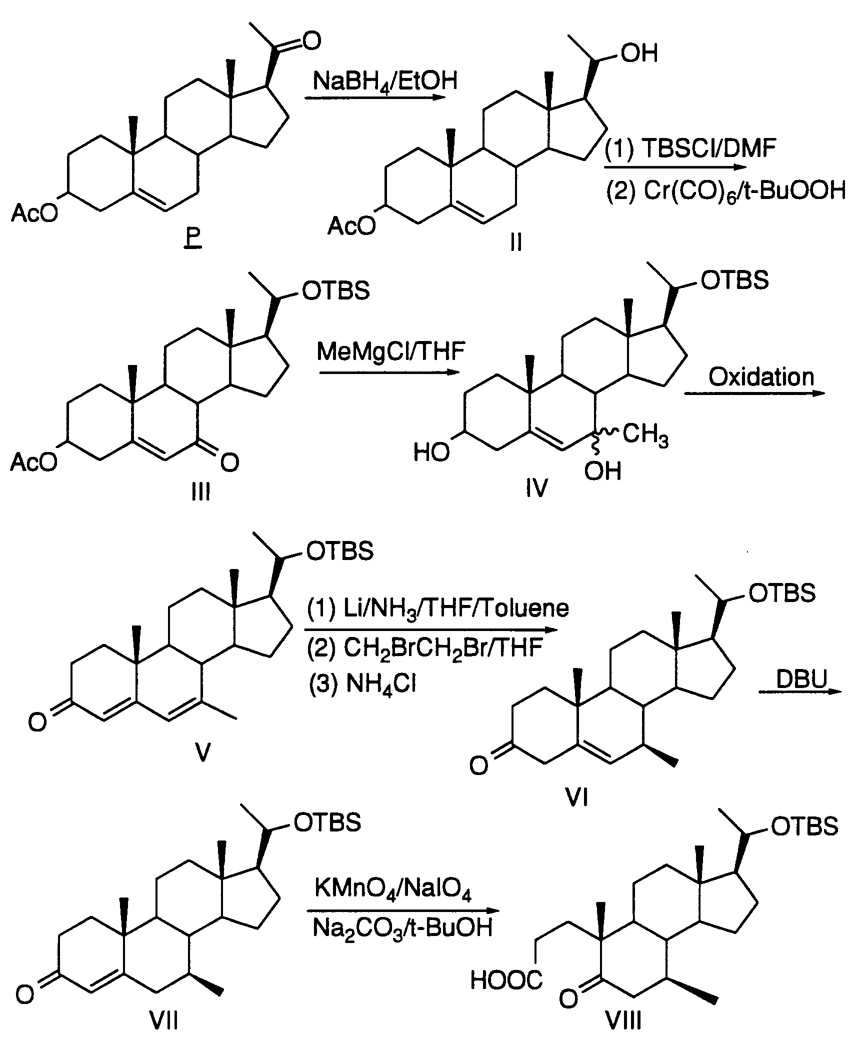

### FLOWSHEET B

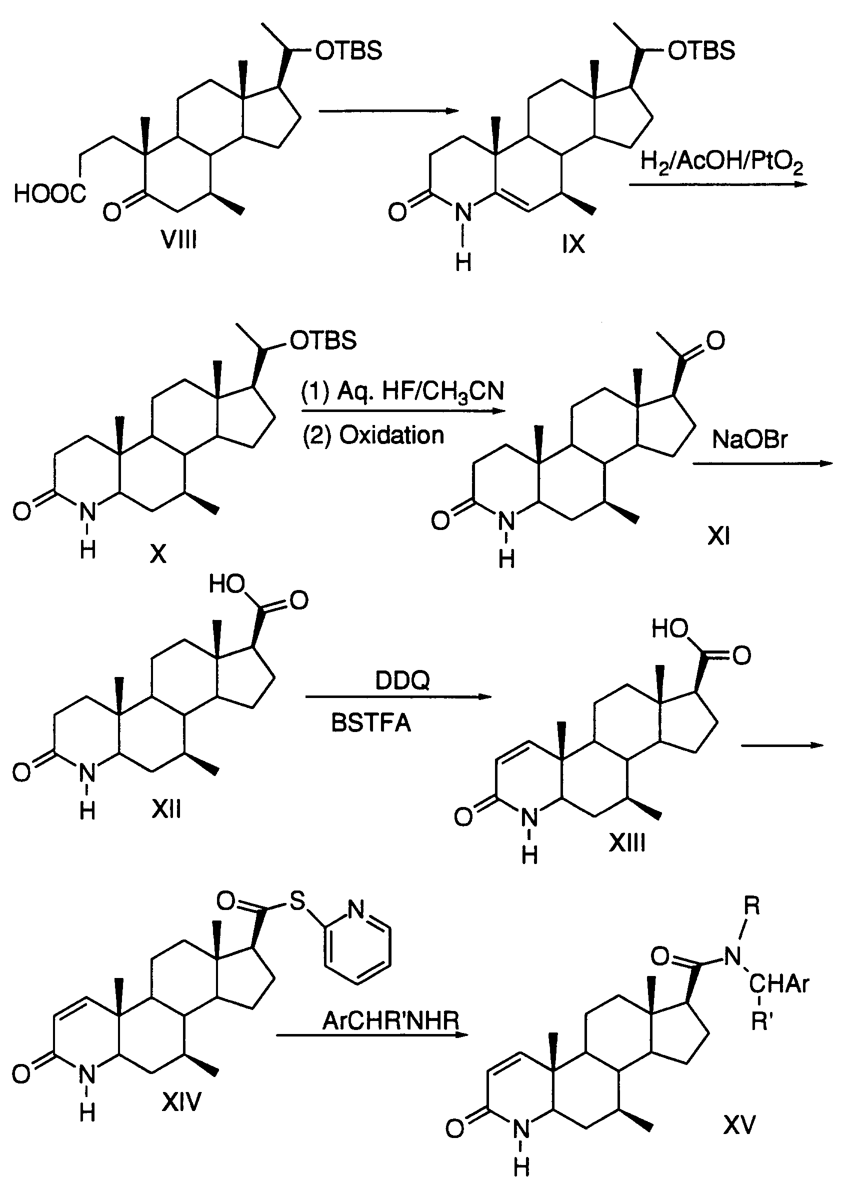

### Biological Assays

### Preparation of Human prostatic and scalp 5α-reductases

Samples of human tissue were pulverized using a freezer mill and homogenized in 40 mM potassium phosphate, pH 6.5, 5 mM magnesium sulfate, 25 mM potassium chloride, 1 mM phenylmethylsulfonyl fluoride, 1 mM dithiothreitol (DTT) containing 0.25 M sucrose using a Potter-Elvehjem homogenizer. A crude nuclear pellet was prepared by centrifugation of the homogenate at 1,500 x g for 15 min. The crude nuclear pellet was washed two times and resuspended in two volumes of buffer. Glycerol was added to the resuspended pellet to a final concentration of 20%. The enzyme suspension was frozen in aliquots at -80°C. The prostatic and scalp reductases were stable for at least 4 months when stored under these conditions.

### 5α-reductase assay

The reaction mixture for the type 1 5α-reductase contained 40 mM potassium phosphate, pH 6.5, 5 mM [7-³H]-testosterone, 1 mM dithiothreitol and 500 µM NADPH in a final volume of 100 µl. The reaction mixture for the type 2 5α-reductase contained 40 mM sodium citrate, pH 5.5, 0.3 mM [7-³H]-testosterone, 1 mM dithiothreitol and 500 µM NADPH in a final volume of 100 µl. Typically, the assay was initiated by the addition of 50-100 µg prostatic homogenate or 75-200 µg scalp homogenate and incubated at 37°C. After 10-50 min. the reaction was quenched by extraction with 250 µl of a mixture of 70% cyclohexane: 30% ethyl acetate containing 10 µg each DHT and T. The aqueous and organic layers were separated by centrifugation at 14,000 rpm in an Eppendorf microfuge. The organic layer was subjected to normal phase HPLC (10 cm Whattman partisil 5 silica column equilibrated in 1 ml/min 70% cyclohexane: 30% ethyl acetate; retention times: DHT, 6.8-7.2 min.; androstanediol, 7.6-8.0 min.; T, 9.1-9.7 min). The HPLC system consisted of a Waters Model 680 Gradient System equipped with a Hitachi Model 655α autosampler, Applied Biosystems Model 757 variable UV detector, and a Radiomatic Model A120 radioactivity analyzer. The conversion of T to DHT was monitored using the radioactivity flow detector by mixing the HPLC effluent with one volume of Flo Scint 1 (Radiomatic). Under the conditions described, the production of DHT was linear for at least 25 min. The only steroids observed with the human prostate and scalp preparations were T, DHT and androstanediol.

### Inhibition studies

Compounds were dissolved in 100% ethanol. IC₅₀ values represent the concentration of inhibitor required to decrease enzyme activity to 50% of the control. IC₅₀ values were determined using a 6 point titration where the concentration of the inhibitor was varied from 0.1 to 1000 nM. Representative compounds of this invention were tested in the above desribed assay for 5α-reductase type 1 and type 2 inhibition.

A compound referred to herein as a 5α-reductase 2 inhibitor is a compound that shows inhibition of the 5α-reductase 2 isozyme in the above-described assay, having an IC₅₀ value of about or under 100 nM.

A compound referred to herein as a 5α-reductase 1 inhibitor is a compound that shows inhibition of the 5α-reductase 1 isozyme in the above-described assay, having an IC₅₀ value of about or under 100 nM.

### Human Dermal Papilla Cell Assay

The dermal papilla is a small group of cells at the base of each hair follicle, and it is presently thought that these cells are stem cells that form the basis for hair growth. These cells have been shown to have 5 alpha reductase activity, and it is therefore possible to test inhibitors of 5 alpha reductase in these cell culture systems.

Isolated and cultured dermal papilla cells are prepared according to the methods of Messenger, A.G., "The Culture of Dermal Papilla Cells From Human Hair Follicles", *Br. J. Dermatol. 110:685*-689, **1984** and Itami, *S. et.al*., "5α-Reductase Activity In Cultured Human Dermal Papilla Cells From Beard Compared With Reticular Dermal Fibroblasts", *J. Invest. Dermatol. 94*:150-152, **1990**. Beard dermal papilla cells and occipital scalp hair of two different individuals are used throughout the study. All experiments are performed at confluency after the fourth to sixth subculture. Confluent monolayers are rinsed twice with phosphate-buffered saline, scraped from dishes by rubber policemen, and collected into a centrifuge tube. The cell suspensions are centrifuged at 1,500 rpm for 10 min at 4°C. The pellets are resuspended in 20 mM Tris-HCl buffer, pH 7.5, at 4°C, containing 250 mM sucrose, 1 mM MgCl₂, and 2 mM CaCl₂, by vortexing and 10 passes through a 25-gauge needle. The crude homogenate is further homogenized by a teflon-glass homogenizer, and is used as the cell homogenate. For the study of subcellular localization of 5α-reductase, the cell homogenate is centrifuged at 800 x g for 10 min to yield a crude nuclear pellet. The resultant supernatant is centrifuged at 10,000 x g for 15 min to produce a crude mitochondrial pellet. The supernatant is centrifuged at 100,000 x g for 60 min to yield a microsomal pellet and cytosol. Each particulate fraction is washed twice and resuspended in the buffer.

A standard incubation mixture will consist of 50 nM [³H]-testosterone, 1 mM NADPH, 100 mM sodium citrate, pH 5.5 or 100 mM Tris-HCl, pH 7.5, and 50 ml of the cell homogenate, in a final volume of 100 ml. Each tube contains 50-100 mg of cellular protein. Incubation is carried out at 37°C for 30 min. During this incubation, the reaction is proportional to the time. For the study of optimum pH, citrate buffer is used at pH 4.5-6.5, and the Tris HCl buffer at pH 7.0-9.0. The protein content is determined by the method of Lowry, *et al*., "Protein Measurement With The Folin Phenol Reagent" *J. Biol. Chem*.. *193*:265-275, **1951**.

After incubation, the reaction is stopped by adding 4 times volume of chloroform-methanol (2/1:V/V) containing 110 mg each of carrier steroids. The extracted steroids are analyzed by thin-layer chromatography as previously described by Gomez, *et al.*, "In Vitro Metabolism Of Testosterone-4-¹⁴C and D-androstene-3, 17-dione-4-¹⁴C In Human Skin" *Biochem., 7*:24-32, **1968**, and the purity of each steroid is determined by the recrystallization method. The activity of 5α-reductase is expressed by the sum of dihydrotestosterone, androstanediol and androstanedione formed. [1,2-³H]-testosterone (55.2 Ci/mmol) is obtainable from New England Nuclear Corporation (Boston, MA) and unlabeled steroids can be purchased from Sigma Chemical Company (St. Louis, MO). Fetal calf serum is obtainable from Hazleton (Lenaxa, Kansas). All other chemicals are of reagent grade.

### Fuzzy Rat Acne Model

Adult fuzzy rats are a variety of rat that has stunted hair growth, brown colored seborrhea covering their entire back skin and abnormally increased sebum production after puberty that has been demonstrataed to be due to circulating androgens. 0.1, 0.05 and 0.025% solutions of a selected 5α-reductase inhibitor of interest are prepared in a vehicle of propylene glycol, isopropanol, isopropyl myristate and water (50/30/2/18%), and is topically applied onto the backs of adult male fuzzy rats, 0.2 ml per animal daily for 4 weeks. Controls receive the vehicle alone and 5 of them are castrated. After 2 weeks seborrhea will be dose-dependently depleted and after 4 weeks bromodeoxyuridine (BrdU, 200 mg/kg) is intraperitoneally injected 2 hours before sacrifice. The skin tissues are incubated with EDTA (20 mM) in phosphate buffer, 1.5 hours at 37°C. The pilo-sebaceous unit attached to the epidermis is striped from the dermis and fixed with formalin for immuno-staining of BrdU. DNA synthesis cells showing a BrdU-positive nucleus are located in the outer glandular border. The number of S-phase cells per lobe is determined with a micro-image apparatus. Using formalin fixed skin, frozen serial sections are stained with 1% osmium and the size of the lobes is measured. A positive inhibitor of skin 5α-reductrase will induce suppression of sebum production by inhibiting the rate of glandular cell turnover, and showing reduced lobular size.

The following describes an example methodology that can be used for detection of hair growth.

### MACROPHOTOGRAPHY AND GLOBAL PHOTOGRAPHY PROCEDURE FOR DETECTION OF HAIR GROWTH

### A. Macrophotographic Procedure

- Location:: ID card Haircount target area
- Equipment:: Film: Kodak-T-max 24 exposure each of same emulsion lot number
- Camera:: Nikon N-6000
- Lens:: Nikkor 60 mm f2.8
- Flashes:: Nikon SB-21B Macroflash
- Device:: registration device

### Photographic Procedure:

In these clinical photographs, the only variable allowed is the haircount. Film emulsion, lighting, framing, exposure, and reproduction ratios are held constant.
1. The haircount area on the patient is prepared as follows: A small (∼1mm) dot tattoo is placed at the beginning of the study at the leading edge of the bald area directly interior to the center of the vertex bald spot, using a commercial tattooing machine or manually (needle and ink). An area approximately one square inch in size, centered at the tattoo at the leading edge of the balding area, is clipped short (∼2mm). Cut hairs are removed from the area to be photographed, using tape. Compressed air and/or ethanol wipes may also be used to facilitate removal of cut hairs.
2. Magnification: Each lens supplied has a fixed reproduction ratio of 1:1.2. Aperture: Every photograph is taken at f/22. Film: T-Max 100 (24 exposure) is used.
3. Patient's haircount target area. Three exposures (-2/3, 0, and +2/3 f-stop).

### B. Global Photographic Procedure

- Locations:: Color card/patient Id Global photograph
- Equipment:: Film: Kodachrome KR-64 24 exposure each of same emulsion lot number
- Camera:: Nikon N-6000
- Lens:: Nikkor 60 mm f2.8
- Flashes:: Nikon SB-23
Color card/patient Id

### Photographic Procedure

In these clinical photographs, the only variable allowed is the global area's appearance. Anything extraneous to the area (clothing, furniture, walls, etc.) is eliminated from the fields to be photographed.
1. Patients will have global photographs taken prior to hair clipping with the head in a fixed position (determined by the supplied stereotactic device). Hair on the patient's head is positioned consistently so as to not obscure the bald area.
2. Magnification: Each lens supplied has a fixed reproduction ratio of 1:6. Aperture: Every photograph will be taken at f/11. Film: Kodachrome (24 exposure) is used.
3. Patient's global photographs. Three exposures at zero compensation.

A trained technician places a transparency over the photograph and, using a felt tip pen, places a black dot over each visible hair. The dot map transparency is then counted using image analysis with computer assistance.

Photographs are coded with a random number corresponding to study site, visit number and patient allocation number to insure blinding to time. At Month 6, baseline and Month 6 photographs are counted and data analyzed for interim analysis. At Month 12, baseline, Month 6 and Month 12 photographs are counted and data analyzed for the primary endpoint.

Methodology for detection of hair growth is also described in Olsen, E.A. and Delong, E., *J. American Academy of Dermatology*, *Vol. 23, p. 470* (**1990**).

## Claims

1. A compound of the Formula I 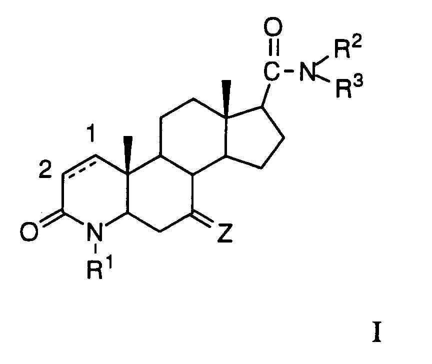 or a pharmaceutically acceptable salt or ester thereof, wherein: the C₁-C₂ bond designated "--------" represents a single or double bond;
R¹ is selected from:
1) -H,
2) -CH₃ and
3) -CH₂CH₃;
Z is selected from:
1) oxo,
2) α-H and a β-substituent selected from:
a) C₁₋₄ alkyl,
b) C₂₋₄ alkenyl,
c) -CH₂ COOH,
d) -OH,
e) -COOH,
f) -COO (C₁₋₄ alkyl),
g) -OCONR⁴R⁵ wherein
R⁴ and R⁵ are independently selected from
i) -H,
ii) -C₁₋₄ alkyl,
iii) phenyl and
iv) benzyl;
or R⁴ and R⁵ taken together with the nitrogen to which they are attached represent a 5-6 membered saturated heterocycle optionally containing one other heteroatom selected from -O-,-NH- and -S-;
h) C₁₋₄ alkoxy,
i) C₃₋₆ cycloalkoxy,
j) -OC(O)R⁷, wherein R⁷ is C₁₋₆alkyl or phenyl,
k) halo,
l) halo-C₁₋₂ alkyl,
m) -CF₃, and
n) C₃₋₆ cycloalkyl;
3) =CHR⁶;
4) spirocyclopropane either unsubstituted or substituted with R⁶;
R² is selected from -H and C₁₋₆ alkyl, either unsubstituted or substituted with one or more of aryl, heteroaryl, -COOH or -OH;
R³ is -C₁₋₆ alkyl substituted with one or more of aryl, heteroaryl, -COOH, -OH or di-aryl amino; and
R⁶ is selected from -H and C₁₋₄ alkyl;
the aryl and heteroaryl moieties referred to above being either unsubstituted or mono- or di-substituted by substituents which are independently selected from: C₁₋₆ alkyl, C₂₋₆ alkenyl, phenyl, halo such as chloro, bromo, iodo and fluoro, trifluoromethyl, cyano, carboxy, C₁₋₆ alkyloxycarbonyl, hydroxy, C₁₋₆ alkoxy, C₁₋₆ alkylcarbonyloxy, C₁₋₆ alkylthio, C₁₋₆ alkylsulfinyl, C₁₋₆ alkylsulfonyl, C₁₋₆ alkylcarbonyl, amino, C₁₋₆ alkylamino, di(C₁₋₆ alkyl)amino, C₁₋₆ alkylcarbonylamino, C₁₋₆ alkyloxycarbonylamino, C₁₋₆ alkylsulfonyl-amino and C₁₋₆ alkylaminosulfonyl.

2. The compound of Claim 1 wherein R¹ is hydrogen or methyl; R² is hydrogen, methyl, ethyl or propyl; Z is an alpha hydrogen and a beta substituent selected from C₁₋₄ alkyl, C₂₋₄ alkenyl, C₁₋₄ alkoxy, halo-C₁₋₂ alkyl, -CF₃, and C₃₋₆ cycloalkyl; and R³ is C₁₋₄ alkyl di-substituted with phenyl, wherein each phenyl ring is independently unsubstituted or mono- or disubstituted and the substituents are independently selected from: C₁₋₆ alkyl, C₂₋₆ alkenyl, phenyl, halo such as chloro, bromo, iodo and fluoro, trifluoromethyl, cyano, carboxy, C₁₋₆ alkyloxycarbonyl, hydroxy, C₁₋₆ alkoxy, C₁₋₆ alkylcarbonyloxy, C₁₋₆ alkylthio, C₁₋₆ alkylsulfinyl, C₁₋₆ alkylsulfonyl, C₁₋₆ alkylcarbonyl, amino, C₁₋₆ alkylamino, di(C₁₋₆ alkyl)amino, C₁₋₆ alkylcarbonylamino, C₁₋₆ alkyloxycarbonylamino, C₁₋₆ alkylsulfonyl-amino and C₁₋₆ alkylaminosulfonyl.

3. The compound of Claim 2 having structural Formula II: 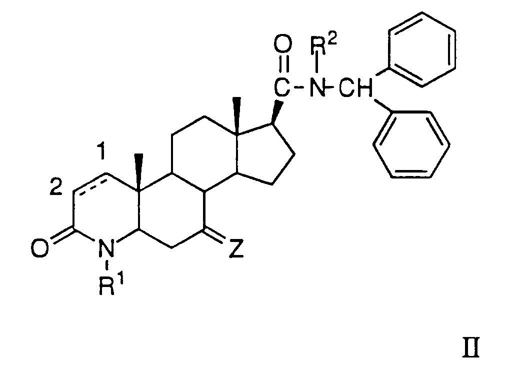 or a pharmaceutically acceptable salt thereof wherein R¹ is hydrogen or methyl; R² is hydrogen or methyl, and Z is alpha hydrogen and beta methyl.

4. The compound of Claim 1 selected from the group consisting of:
N-(diphenylmethyl)-3-oxo-4-aza-7β-methyl-5α-androstane-17β-carboxamide;
N-(diphenylmethyl)-3-oxo-4-aza-7β-methyl-5α-androst-1-ene-17β-carboxamide;
N-(diphenylmethyl)-3-oxo-4-aza-4,7β-dimethyl-5α-androstane-17β-carboxamide; and
N-(methyl),N-(diphenylmethyl)-3-oxo-4-aza-4,7β-dimethyl-5α-androstane-17β-carboxamide;
or a pharmaceutically acceptable salt thereof.

5. The use of a compound of Claim 1 for the manufacture of a medicament for inhibiting 5α-reductase or the isozymes thereof.

6. The use of a compound of Claim 1 for the manufacture of a medicament for treating acne vulgaris, androgenic alopecia, female hirsutism, benign prostatic hyperplasia, prostatitis, and the treatment and/or prevention of prostatic cancer.

7. The use as claimed in Claim 6 wherein the androgenic alopecia is male pattern alopecia.

8. The use of a compound of Claim 1 for the manufacture of a medicament for arresting and reversing androgenic alopecia and promoting hair growth.

9. The use as claimed in Claim 8 wherein the androgenic alopecia is male pattern alopecia.

10. The use of a compound of Claim 1 in combination with an inhibitor of 5α-reductase 1 and/or an inhibitor of 5α-reductase 2 for the manufacture of a medicament for inhibiting 5α-reductase or the isozymes thereof.

11. The use of a compound of Claim 1 in combination with an inhibitor of 5α-reductase 1 and/or an inhibitor of 5α-reductase 2 for the manufacture of a medicament for treating acne vulgaris, androgenic alopecia, female hirsutism, benign prostatic hyperplasia, prostatitis, and the treatment and/or prevention of prostatic cancer.

12. The use as claimed in Claim 11 wherein the inhibitor of 5α-reductase 2 is finasteride.

13. The use of a compound of Claim 1 in combination with an inhibitor of 5α-reductase 1 and/or an inhibitor of 5α-reductase 2 for the manufacture of a medicament for arresting and reversing androgenic alopecia and promoting hair growth.

14. The use of a compound of Claim 1 in combination with a potassium channel opener for the manufacture of a medicament for treating androgenic alopecia.

15. The use as claimed in Claim 14 wherein the androgenic alopecia is male pattern alopecia.

16. The use as claimed in Claim 14 wherein the potassium channel opener is minoxidil or a pharmaceutically acceptable salt thereof.

17. The use of a compound of Claim 1 in combination with a potassium channel opener for the manufacture of a medicament for arresting and reversing androgenic alopecia and promoting hair growth.

18. A pharmaceutical composition comprising a pharmaceutically acceptable carrier and a therapeutically effective amount of a compound of Claim 1.

19. A pharmaceutical composition comprising a pharmaceutically acceptable carrier and a therapeutically effective amount of a compound of Claim 1 and a compound selected from finasteride and minoxidil, or a pharmaceutically acceptable salt thereof.

20. A pharmaceutical composition comprising a pharmaceutically acceptable carrier adapted for topical application and a therapeutically effective amount of a compound of Claim 1 and a compound selected from finasteride and minoxidil, or a pharmaceutically acceptable salt thereof.

21. A pharmaceutical composition comprising a pharmaceutically acceptable carrier adapted for oral administration and a therapeutically effective amount of a compound of Claim 1 and finasteride or a pharmaceutically acceptable salt thereof.

## Patentansprüche

1. Eine Verbindung der Formel I 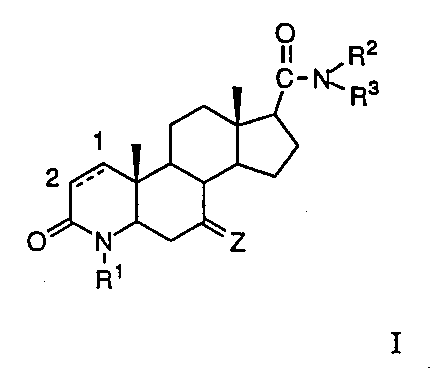 oder ein pharmazeutisch annehmbares Salz oder ein pharmazeutisch annehmbarer Ester davon, worin:
die mit "--------" gekennzeichnete C₁-C₂-Bindung eine Einfach- oder Doppelbindung darstellt;
R¹ ausgewählt ist aus:
1) -H,
2) -CH₃ und
3) -CH₂CH₃;
Z ausgewählt ist aus:
1) Oxo,
2) α-H und einem β-Substituenten, der ausgewählt ist aus:
a) C₁₋₄-Alkyl,
b) C₂₋₄-Alkenyl,
c) -CH₂COOH,
d) -OH,
e) -COOH,
f) -COO(C₁₋₄-Alkyl),
g) -OCONR⁴R⁵, worin
R⁴ und R⁵ unabhängig voneinander ausgewählt sind aus
i) -H,
ii) -C₁₋₄-Alkyl,
iii) Phenyl und
iv) Benzyl,
oder R⁴ und R⁵, zusammengenommen mit dem Stickstoff, an den sie gebunden sind, einen 5-6gliedrigen gesättigten Heterocyclus darstellen, der gegebenenfalls ein anderes Heteroatom, das ausgewählt ist aus -O-, -NH- und -S-, enthält,
h) C₁₋₄-Alkoxy,
i) C₃₋₆-Cycloalkoxy,
j) -OC(O)R⁷, worin R⁷ C₁₋₆-Alkyl oder Phenyl ist,
k) Halogen,
l) Halogen-C₁₋₂-alkyl,
m) -CF₃ und
n) C₃₋₆-Cycloalkyl;
3) =CHR⁶,
4) Spirocyclopropan, entweder unsubstituiert oder substituiert mit R⁶;
R² ausgewählt ist aus -H und C₁₋₆-Alkyl, entweder unsubstituiert oder substituiert mit einem oder mehreren Aryl, Heteroaryl, -COOH oder -OH;
R³ -C₁₋₆-Alkyl ist, substituiert mit einem oder mehreren Aryl, Heteroaryl, -COOH, -OH oder Diarylamino; und
R⁶ ausgewählt ist aus -H und C₁₋₄-Alkyl;
wobei die Aryl- und Heteroarylreste, auf die oben Bezug genommen wird, entweder unsubstituiert oder mono- oder disubstituiert sind mit Substituenten, die unabhängig ausgewählt sind aus: C₁₋₆-Alkyl, C₂₋₆-Alkenyl, Phenyl, Halogen, wie z.B. Chlor, Brom, Jod und Fluor, Trifluormethyl, Cyano, Carboxy, C₁₋₆-Alkyloxycarbonyl, Hydroxy, C₁₋₆-Alkoxy, C₁₋₆-Alkylcarbonyloxy, C₁₋₆-Alkylthio, C₁₋₆-Alkylsulfinyl, C₁₋₆-Alkylsulfonyl, C₁₋₆-Alkylcarbonyl, Amino, C₁₋₆-Alkylamino, Di(C₁₋₆-alkyl)amino, C₁₋₆-Alkylcarbonylamino, C₁₋₆-Alkyloxycarbonylamino, C₁₋₆-Alkylsulfonylamino und C₁₋₆-Alkylaminosulfonyl.

2. Die Verbindung nach Anspruch 1, worin R¹ Wasserstoff oder Methyl ist; R² Wasserstoff, Methyl, Ethyl oder Propyl ist; Z ein alpha-Wasserstoff und ein beta-Substituent ist, der ausgewählt ist aus C₁₋₄-Alkyl, C₂₋₄-Alkenyl, C₁₋₄-Alkoxy, Halogen-C₁₋₂-alkyl, -CF₃ und C₃₋₆-Cycloalkyl; und R³ mit Phenyl disubstituiertes C₁₋₄-Alkyl ist, wobei jeder Phenylring unabhängig unsubstituiert oder mono- oder disubstituiert ist und die Substituenten unabhängig ausgewählt sind aus: C₁₋₆-Alkyl, C₂₋₆-Alkenyl, Phenyl, Halogen, wie z.B. Chlor, Brom, Iod und Fluor, Trifluormethyl, Cyano, Carboxy, C₁₋₆-Alkyloxycarbonyl, Hydroxy, C₁₋₆-Alkoxy, C₁₋₆-Alkylcarbonyloxy, C₁₋₆-Alkylthio, C₁₋₆-Alkylsulfinyl, C₁₋₆-Alkylsulfonyl, C₁₋₆-Alkylcarbonyl, Amino, C₁₋₆-Alkylamino, Di(C₁₋₆-alkyl)amino, C₁₋₆-Alkylcarbonylamino, C₁₋₆-Alkyloxycarbonylamino, C₁₋₆-Alkylsulfonylamino und C₁₋₆-Alkylaminosulfonyl.

3. Die Verbindung nach Anspruch 2, die die Strukturformel II hat: 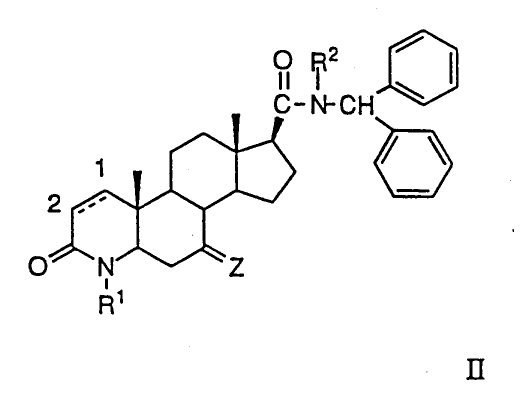 oder ein pharmazeutisch annehmbares Salz davon, worin
R¹ Wasserstoff oder Methyl ist; R² Wasserstoff oder Methyl ist und Z alpha-Wasserstoff und beta-Methyl ist.

4. Die Verbindung nach Anspruch 1, ausgewählt aus der Gruppe, die besteht aus:
N-(Diphenylmethyl)-3-oxo-4-aza-7β-methyl-5α-androstan-17β-carboxamid;
N-(Diphenylmethyl)-3-oxo-4-aza-7β-methyl-5α-androst-1-en-17β-carboxamid;
N-(Diphenylmethyl)-3-oxo-4-aza-4,7β-dimethyl-5α-androstan-17β-carboxamid und
N-(Methyl),N-(diphenylmethyl)-3-oxo-4-aza-4,7β-dimethyl-5α-androstan-17β-carboxamid;
oder ein pharmazeutisch annehmbares Salz davon.

5. Die Verwendung einer Verbindung nach Anspruch 1 für die Herstellung eines Medikaments zur Inhibierung von 5α-Reduktase oder ihren Isoenzymen.

6. Die Verwendung einer Verbindung nach Anspruch 1 für die Herstellung eines Medikaments zur Behandlung von Akne vulgaris, androgener Alopezie, weiblichem Hirsutismus, benigner Prostatahyperplasie, Prostatitis und zur Behandlung und/oder Prävention von Prostatakrebs.

7. Die wie in Anspruch 6 beanspruchte Verwendung, wobei die androgene Alopezie Alopezie des männlichen Typs ist.

8. Die Verwendung einer Verbindung nach Anspruch 1 für die Herstellung eines Medikamente zum Stoppen und zur Umkehrung von androgener Alopezie und zur Förderung von Haarwachstum.

9. Die wie in Anspruch 8 beanspruchte Verwendung, wobei die androgene Alopezie Alopezie des männlichen Typs ist.

10. Die Verwendung einer Verbindung nach Anspruch 1 in Verbindung mit einem Inhibitor von 5α-Reduktase 1 und/oder einem Inhibitor von 5α-Reduktase 2 für die Herstellung eines Medikamente zur Inhibierung von 5α-Reduktase oder ihren Isoenzymen.

11. Die Verwendung einer Verbindung nach Anspruch 1 in Verbindung mit einem Inhibitor von 5α-Reduktase 1 und/oder einem Inhibitor von 5α-Reduktase 2 für die Herstellung eines Medikamente zur Behandlung von Akne vulgaris, androgener Alopezie, weiblichem Hirsutismus, benigner Prostatahyperplasie, Prostatitis und für die Behandlung und/oder Prävention von Prostatakrebs.

12. Die wie in Anspruch 11 beanspruchte Verwendung, wobei der Inhibitor von 5α-Reduktase 2 Finasterid ist.

13. Die Verwendung einer Verbindung nach Anspruch 1 in Verbindung mit einem Inhibitor von 5α-Reduktase 1 und/oder einem Inhibitor von 5α-Reduktase 2 für die Herstellung eine Medikamente zum Stoppen und zur Umkehrung von androgener Alopezie und zur Förderung von Haarwachstum.

14. Die Verwendung einer Verbindung nach Anspruch 1 in Verbindung mit einem Kaliumkanalöffner für die Herstellung eines Medikaments zur Behandlung von androgener Alopezie.

15. Die wie in Anspruch 14 beanspruchte Verwendung, wobei die androgene Alopezie Alopezie des männlichen Typs ist.

16. Die wie in Anspruch 14 beanspruchte Verwendung, wobei der Kaliumkanalöffner Minoxidil oder ein pharmazeutisch annehmbares Salz davon ist.

17. Die Verwendung einer Verbindung nach Anspruch 1 in Verbindung mit einem Kaliumkanalöffner für die Herstellung eines Medikaments zum Stoppen und zur Umkehrung von androgener Alopezie und zur Förderung von Haarwachstum.

18. Eine pharmazeutische Zusammensetzung, die einen pharmazeutisch annehmbaren Träger und eine therapeutisch wirksame Menge einer Verbindung nach Anspruch 1 enthält.

19. Eine pharmazeutische Zusammensetzung, die einen pharmazeutisch annehmbaren Träger und eine therapeutisch wirksame Menge einer Verbindung nach Anspruch 1 und eine Verbindung, die ausgewählt ist aus Finasterid und Minoxidil, oder ein pharmazeutisch annehmbares Salz davon enthält.

20. Eine pharmazeutische Zusammensetzung, die einen pharmazeutisch annehmbaren Träger, der für die topische Anwendung angepaßt ist, und eine therapeutisch wirksame Menge einer Verbindung nach Anspruch 1 und eine Verbindung, die ausgewählt ist aus Finasterid und Minoxidil, oder ein pharmazeutisch annehmbares Salz davon enthält.

21. Eine pharmazeutische Zusammensetzung, die einen pharmazeutisch annehmbaren Träger, der für die orale Verabreichung angepaßt ist, und eine therapeutisch wirksame Menge einer Verbindung nach Anspruch 1 und Finasterid oder ein pharmazeutisch annehmbares Salz davon enthält.

## Revendications

1. Composé de formule 1 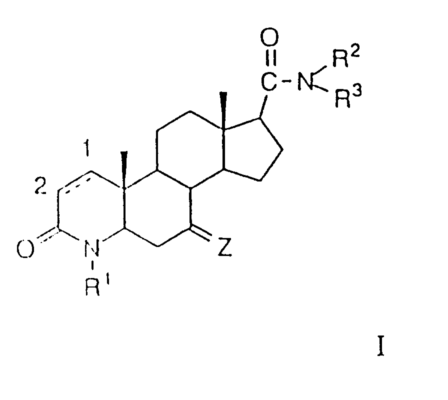 ou un sel ou ester de celui-ci acceptable en pharmacie, où : La liaison C₁-C₂ désignée "--------" représente une liaison simple ou double;
R¹ est choisi parmi :
1) -H,
2) -CH₃ et
3) -CH₂CH₃;
Z est choisi parmi :
1) l'oxo,
2) le α-H et un β-substituant choisi parmi :
a) un C₁₋₄alkyle,
b) un C₂₋₄alcényle,
c) -CH₂COOH,
d) -OH,
e) -COOH,
f) -COO(C₁₋₄alkyl),
g) -OCONR⁴R⁵ où
R⁴ et R⁵ sont choisis, de façon indépendante, parmi
i) -H,
ii) -C₁₋₄alkyl,
iii) le phényle et
iv) le benzyle;
ou R⁴ et R⁵ pris ensemble avec l'azote auquel ils sont attachés représentent un hétérocycle saturé à 5 ou 6 chaînons contenant, si on le désire, un autre hétéroatome choisi parmi -O-, -NH- et -S-;
h) un C₁₋₄alcoxy,
i) un C₃₋₆cycloalcoxy,
j) -OC(O)R⁷, où R⁷ est un C₁₋₆alkyle ou le phényle,
k) un halo,
l) un halo-C₁₋₂alkyl,
m) -CF₃, et
n) un C₃₋₆cycloalkyle;
3) =CHR⁶;
4) un spirocyclopropane soit non substitué soit substitué avec R⁶;
R² est choisi parmi -H et un C₁₋₆alkyle, soit non substitué soit substitué avec au moins un aryle, hétéroaryle, -COOH ou -OH;
R³ est -C₁₋₆alkyl substitué avec au moins un aryle, hétéroaryle, -COOH, -OH ou diarylamino ; et
R⁶ est choisi parmi -H et un C₁₋₄alkyle;
les portions aryles et hétéroaryles mentionnées ci-dessus étant soit non substituées, soit mono- ou disubstituées par des substituants qui sont choisis, de façon indépendante, parmi : un C₁₋₆alkyle, un C₂₋₆alcényle, le phényle, un halo comme le chloro, le bromo, l'iodo et le fluoro, le trifluorométhyle, le cyano, le carboxy, un C₁₋₆alkyloxycarbonyle, l'hydroxy, un C₁₋₆alcoxy, un C₁₋₆alkylcarbonyloxy, un C₁₋₆alkylthio, un C₁₋₆alkylsulfinyle, un C₁₋₆alkylsulfonyle, un C₁₋₆alkylcarbonyle, un amino, un C₁₋₆alkylamino, un di(C₁₋₆alkyl)amino, un C₁₋₆alkylcarbonylamino, un C₁₋₆alkyloxycarbonylamino, un C₁₋₆alkylsulfonyl-amino et un C₁₋₆alkylaminosulfonyle.

2. Composé selon la revendication 1 où R¹ est l'hydrogène ou le méthyle ; R² est l'hydrogène, le méthyle, l'éthyle ou le propyle ; Z est un hydrogène en alpha et un substituant en bêta choisi parmi un C₁₋₄alkyle, un C₂₋₄alcényle, un C₁₋₄alcoxy, un halo-C₁₋₂alkyl, -CF₃ et un C₃₋₆cycloalkyle ; et R³ est un C₁₋₄alkyle disubstitué avec le phényle, où chaque cycle phényle est, de façon indépendante, non substitué ou mono ou di-substitué, et les substituants sont choisis, de façon indépendante, parmi : un C₁₋₆alkyle, un C₂₋₆alcényle, le phényle, un halo comme le chloro, le bromo, l'iodo et le fluoro, le trifluorométhyle, le cyano, le carboxy, un C₁₋₆alkyloxycarbonyle, l'hydroxy, un C₁₋₆alcoxy, un C₁₋₆alkylcarbonyloxy, un C₁₋₆alkylthio, un C₁₋₆alkylsulfinyle un C₁₋₆alkylsulfonyle, un C₁₋₆alkylcarbonyle, un amino, un C₁₋₆alkylamino, un di(C₁₋₆alkyl)amino, un C₁₋₆alkylcarbonylamino, un C₁₋₆alkyloxycarbonylamino, un C₁₋₆alkylsulfonyl-amino et un C₁₋₆alkylaminosulfonyle.

3. Composé selon la revendication 2 ayant le formule structural II: 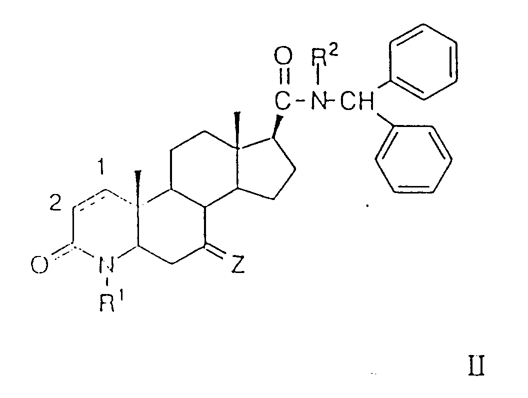 ou un sel de celui-ci acceptable en pharmacie où
R¹ est l'hydrogène ou le méthyle ; R² est l'hydrogène ou le méthyle, et Z est l'hydrogène en alpha et le méthyle en bèta.

4. Composé selon la revendication 1, choisi dans le groupe formé par :
le N-(diphénylméthyl)-3-oxo-4-aza-7β-méthyl-5α-androstane-17β-carboxamide;
le N-(diphénylméthyl)-3-oxo-4-aza-7β-méthyl-5α-androst-1-ène-17β-carboxamide;
le N-(diphénylméthyl)-3-oxo-4-aza-4,7β-diméthyl-5α-androstane-17β-carboxamide ; et
le N-(méthyl),N-(diphénylméthyl)-3-oxo-4-aza-4,7β-diméthyl-5α-androstane-17β-carboxamide;
ou un sel de celui-ci acceptable en pharmacie.

5. Emploi d'un composé selon la revendication 1, pour la fabrication d'un médicament pour inhiber la 5α-réductase ou ses isozymes.

6. Emploi d'un composé selon la revendication 1 pour la fabrication d'un médicament pour traiter l'acné vulgaris, l'alopécie androgénique, l'hirsutisme féminin, l'hyperplasie prostatique bénigme, la prostatite et le traitement et/ou la prévention du cancer prostatique.

7. Emploi tel que revendiqué dans la revendication 6, où l'alopécie antrogénique est l'alopécie masculine localisée.

8. Emploi d'un composé selon la revendication 1 pour la fabrication d'un médicament pour arrêter et inverser l'alopécie androgénique et pour promouvoir la croissance des cheveux.

9. Emploi tel que revendiqué dans la revendication 8, où l'alopécie androgénique est l'alopécie masculine localisée.

10. Emploi d'un composé selon la revendication 1 en combinaison avec un inhibiteur de 5α-réductase 1 et/ou un inhibiteur de 5α-réductase 2 pour la fabrication d'un médicament pour inhiber la 5α-réductase ou ses isozymes.

11. Emploi d'un composé selon la revendication 1 en combinaison avec un inhibiteur de 5α-réductase 1 et/ou un inhibiteur de 5α-réductase 2 pour la fabrication d'un médicament pour traiter l'acné vulgaris, l'alopécie androgénique, l'hirsutisme féminin, l'hyperplasie prostatique bénigme, la prostatite et le traitement et/ou la prévention du cancer prostatique.

12. Emploi tel que revendiqué dans la revendication 11, où l'inhibiteur de 5α-réductase 2 est le finastéride.

13. Emploi d'un composé selon la revendication 1 en combinaison avec un inhibiteur de 5α-réductase 1 et/ou un inhibiteur de 5α-réductase 2 pour la fabrication d'un médicament pour arrêter et inverser l'alopécie androgénique et pour promouvoir la croissance des cheveux.

14. Emploi d'un composé selon la revendication 1 en combinaison avec un ouvreur de canal de potassium pour la fabrication d'un médicament pour traiter l'alopécie androgénique.

15. Emploi tel que revendiqué dans la revendication 14, où l'alopécie androgénique est l'alopécie masculine localisée.

16. Emploi tel que revendiqué dans la revendication 14, où l'ouvreur de canal de potassium est le minoxidil ou un sel de celui-ci acceptable en pharmacie.

17. Emploi d'un composé selon la revendication 1 en combinaison avec un ouvreur de canal de potassium pour la fabrication d'un médicament pour arrêter et inverser l'alopécie androgénique et pour promouvoir la croissance des cheveux.

18. Composition pharmaceutique comportant un support acceptable en pharmacie et une quantité thérapeutiquement efficace d'un composé selon la revendication 1.

19. Composition pharmaceutique comportant un support acceptable en pharmacie et une quantité thérapeutiquement efficace d'un composé selon la revendication 1 et un composé choisi parmi le finastéride et le minoxidil ou un sel de ceux-ci acceptable en pharmacie.

20. Composition pharmaceutique comportant un support acceptable en pharmacie adapté à une application topique et une quantité thérapeutiquement efficace d'un composé de la revendication 1 et un composé choisi parmi le finastéride et le minoxidil ou un sel de ceux-ci acceptable en pharmacie.

21. Composition pharmaceutique comportant un support acceptable en pharmacie adapté à une administration orale et une quantité thérapeutiquement efficace d'un composé de la revendication 1 et le finastéride ou un sel de celui-ci acceptable en pharmacie.
